# EUROPÄISCHE PATENTANMELDUNG

(11) **EP 3 034 601 A2**
(43) Veröffentlichungstag der Anmeldung: **22.06.2016**
(21) Anmeldenummer: 15003615.0
(22) Anmeldetag: 18.12.2015
(51) Int. Cl.: C12M 1/00, C12M 1/34

(54) **ANLAGE UND STEUERUNGSVERFAHREN ZUR ZUCHT VON PHOTOTROPHEN ORGANISMEN**

(30) Priorität: 18.12.2014 DE 102014018697
(71) Anmelder: ERWIN SANDER ELEKTROAPPARATEBAU GMBH, 31311 Uetze-Eltze (DE); Hochschule für Technik und Wirtschaft des Saarlandes, 66117 Saarbrücken (DE)
(72) Erfinder: KULAKOWSKI, Andreas, 29355 Beedenbostel (DE); WALLER, Uwe, 66113 Saarbrücken (DE); SANDER, Martin, 31311 Uetze-Eltze (DE)
(74) Vertreter: Friedrich, Andreas

(57) **Zusammenfassung**

Die Erfindung betrifft eine Vorrichtung zur Zucht von phototrophen Organismen, umfassend
a. einen Reaktorkreislauf (10) mit einer Reaktorpumpe (12),
b. einen Versorgungskreislauf (20) mit einer Versorgungspumpe (22) zum Aufbereiten einer Versorgungsflüssigkeit (24) und zum Versorgen des Reaktorkreislaufs (20) mit der Versorgungsflüssigkeit (24), und
c. eine Dialysemembran (30), die den Versorgungskreislauf (20) von dem Reaktorkreislauf (10) trennt, aber einen diffusiven, durch eine Porengröße der Dialysemembran (30) begrenzten Stoffaustausch erlaubt.

Die Erfindung betrifft ferner ein Verfahren zum Steuern einer Anlage zur Produktion von phototrophen Organismen.

## Beschreibung

Die Erfindung betrifft eine Anlage zur Zucht von phototrophen Organismen sowie ein Verfahren zum Steuern einer solchen Anlage. Als Energielieferant oder Futtermittel ist die Produktion von phototrophen Organismen wie Mikroalgen oder Cyanobakterien von wachsendem Interesse. Aus Kostengründen ist es dabei vorteilhaft, wenn bei anderen Prozessen anfallende Ab- oder Prozesswässer als Nährstoffquellen genutzt werden können. Dies ist ein wichtiger Schritt, um die Wirtschaftlichkeit algenbasierter Kraftstoff- und Futterproduktionen langfristig zu verbessern. Als Limitierung haben sich dabei regelmäßig die relativ geringen Nährstoffkonzentrationen im Prozess- und Abwasser herausgestellt, was bei Anwendung von herkömmlichen Verfahren zu einer geringen Produktivität führt.

Es ist weiterhin bekannt, dass die Produktivität eines Photobioreaktors von der Biomassekonzentration im Inneren des Reaktors abhängt. Die optimale Konzentration hängt wiederum von der einfallenden Lichtstärke ab und ist somit tages- und jahreszeitabhängig, da sie mit dem Stand der Sonne variiert. Eine Anpassung der Biomassekonzentration in Echtzeit ist mit herkömmlichen Verfahren nicht realisierbar. Aus dem Stand der Technik bekannt sind beispielsweise die so genannte Batch-weise Produktion von Mikroalgen sowie verschiedene Durchflussverfahren. Bei der Batch-weisen Produktion wird ein abgeschlossenes Volumen in einem Photobioreaktor zur Zucht der Algen verwendet und zur Algenernte vollständig oder nahezu vollständig aus dem Reaktor entfernt. Im Durchfluss- bzw. Chemostat-Verfahren wird das Wasser zur Nährstoffversorgung der Kultur unbehandelt in den Reaktor eingespeist. Der optimale Durchfluss ist abhängig von der Konzentration der limitierenden Nährstoffkomponente. Der Volumenstrom an ausfließende Algensuspension ist gleich dem einfließenden Volumenstrom. Die Konzentration der limitierenden Nährstoffkomponente bestimmt somit die volumetrische Produktivität. Das Verfahren wurde sowohl mit Teich-Systemen als auch mit Photobioreaktoren erprobt (s. z.B, Abeliovich, A., 2004. Water Pollution and Bioremediation by Microalgae. Water Purification: Algae in Wastewater Oxidation Ponds. In: Richmond A. (Ed.). Handbook of Microalgal Culture: Biotechnology and Applied Phycology. Blackwell Publishing Ltd, Oxford, S. 380ff oder Feng et al, 2011. Lipid production of Chlorella vulgaris cultured in artificial wastewater medium. Bioresource Technology 102, S.101-105).

Ebenfalls bekannt sind Durchflussverfahren, bei denen genutzte Prozess- oder Abwasser zunächst entkeimt werden. Weiterhin ist es bekannt, eine Stufe zur Zellrückhaltung vorzusehen. Dies ist sinnvoll, wenn die Algenkultur nicht übermäßig ausgedünnt werden soll, das zufließende Prozesswasser aber eine relativ geringe Nährstoffkonzentration hat und dementsprechend das Zuführen von Nährstoffen auch einen Abfluss von Volumen aus dem Reaktor oder dem Teich erforderlich macht. Die Algen werden dabei unter Energieaufwand durch mechanische Extraktionsverfahren von der Suspension getrennt und als Konzentrat dem Reaktorvolumen wieder zugeführt. Dieser Prozess ist energieaufwendig und kann mechanischen Stress auf die Algen ausüben. Eine weitere Möglichkeit zur Zeilrückhaltung, die getestet wurde, ist es, die Algensuspension durch eine Polymermembran zu saugen, wobei Flüssigkeit entfernt wird, die Algen jedoch zurückgehalten werden. Eine weitere bekannte Möglichkeit, um Mikroalgenzellen im Reaktormedium zurückzuhalten, ist die Immobilisierung mit Alginat.

Es sind Ansätze bekannt, bei denen Stoffwechselendprodukte und das Wachstum der Algen inhibierende Sekundärmetabolite mittels Dialyse aus dem Reaktorvolumen ausgeschwemmt werden. Eine Erweiterung dieser Methode stellt die sogenannte kontinuierliche Dialyse-Kultur dar, bei der die Dialysat-Seite nicht ausschließlich zum Abtransport von unerwünschten Inhaltsstoffen des Reaktorvolumens, sondern auch zur Nährstoffversorgung der Kultur dient. Hierzu wird Ozeanwasser auf der Dialysat-Seite bereitgestellt, so dass anorganische Nährstoffe zugeführt werden können (s. z.B. Marsot et al., 1991. Growth kinetics and nitrogen-nutrition of the marine diatom Phaeodactylum tricornutum in continuous dialysis culture. Journal of Applied Phycology 3, S. 1-10 und Ney et al., 1981. High density diatom production utilizing dialysis techniques. Aquaculture 24, S.363-369).

Probleme, die bei kommerziell verfügbaren Photobioreaktoren nach wie vor bestehen, sind beispielsweise der Aufwand, der zur Aufrechterhaltung von hygienischen Verhältnissen im Reaktor betrieben werden muss, sowie die ineffiziente Ausnutzung von zur Verfügung stehenden Wässern mit niedriger Nährstoffkonzentration. Die derzeit genutzten Verfahren zur Zellretention und somit zur Erhöhung der optischen Dichte im Reaktorvolumen sind energieaufwendig und belasten die gezüchteten Organismen. Ohnehin ist es wünschenswert, die optische Dichte der Zellsuspension auf einfache Art und Weise an die zur Verfügung stehende Lichtmenge anzupassen. Der vorliegenden Erfindung liegt daher die Aufgabe zugrunde, Nachteile im Stand der Technik zu beseitigen.

Gemäß einem ersten Aspekt löst die Erfindung die Aufgabe durch eine Anlage zur Zucht von phototrophen Organismen der eingangs erwähnten Art, die einen Versorgungskreislauf mit einer Versorgungspumpe zum Aufbereiten einer Versorgungsflüssigkeit und zum Versorgen des Reaktorkreislaufs mit der Versorgungsflüssigkeit sowie eine Dialysemembran aufweist, die den Versorungskreislauf von dem Reaktorkreislauf trennt, aber einen diffusiven, durch eine Porengröße der Dialysemembran begrenzten Stoffaustausch erlaubt.

Gemäß einem zweiten Aspekt löst die Erfindung das Problem durch ein Verfahren zum Steuern einer Anlage zur Produktion von phototrophen Organismen mit den Schritten:
a) Bestimmen eines Kennwerts für eine auf einen Reaktor zur Zucht von phototrophen einfallenden Lichtmenge
b) Bestimmen eines Sollwerts für ein Volumen in einem Zuchtbehälter unter Einbeziehung des Kennwerts für die einfallende Lichtmenge und/oder der Uhrzeit, und
c) Anpassen einer Pumpleistung einer Reaktorpumpe und/oder einer Versorgungspumpe derart, dass der Sollwert des Volumens im Reaktor eingestellt wird.

Die Erfindung ist eine Weiterentwicklung des kontinuierlichen Dialyse-Verfahrens. Im Gegensatz zu bisher verfolgten Ansätzen wird aber kein unbehandeltes Ozeanwasser auf der Dialysat-Seite verwendet, sondern es wird auf Dialysat-Seite ein zweiter Kreislauf aufgebaut, in dem das zur Verfügung gestellte Speisewasser aufbereitet werden kann. Das Vorsehen von zwei über eine Dialysemembran miteinander verbundenen Kreisläufen, die jeweils eine Pumpe aufweisen, bittet weiterhin die Möglichkeit, Flüssigkeit aus dem einen Kreislauf in den anderen Kreislauf zu transferieren und somit die Volumina und Konzentrationen an Nährstoffen, Algen und anderen Inhaltsstoffen zu beeinflussen. Durch den geschlossenen Kreislauf auf Dialysat-Seite bietet sich die Möglichkeit, zur Verfügung stehendes, nährstoffbeinhaltendes Wasser effizient auszunutzen, da das Verfahren nicht darauf angewiesen ist, das bei einem einzigen Durchfluss des Speisewassers alle nötigen Nährstoffe extrahiert werden. Stattdessen kann das Speisewasser im Versorgungskreislauf gelassen werden und erst dann ausgetauscht werden, wenn eine bestimmt Nährstoffkonzentration unterschritten wird.

Durch eine geeignete Steuerung der Pumpen kann ein Transmembranfluss in die eine oder in die andere Richtung eingestellt werden. Es ist somit möglich, das Flüssigkeitsvolumen insbesondere im Reaktorkreislauf einzustellen, ohne Algen aus der Suspension zu entnehmen oder in die Suspension einzubringen. Es ist somit möglich, die Algenkonzentration einzustellen, gleichzeitig wird durch diesen Vorgang die optische Dichte beeinflusst. Diese wiederum ist ein wichtiges Kriterium, um das so genannte photosynthetische Optimum einzustellen. Ist die optische Dichte zu niedrig, so wird nur ein geringer Teil der eingestrahlten Lichtenergie zur Produktion von Biomasse verwendet. Ist die optische Dichte zu hoch, so wird ein relativ großer Teil der eingestrahlten Lichtmenge nicht photosynthetisch verarbeitet, sondern lediglich absorbiert und in Wärmeenergie umgewandelt. Dies ist nachteilig, da ein auf diese Weise überhitzter Reaktor aufwändig gekühlt werden muss. Die einfallende Lichtmenge ist vom Wetter sowie tages- und jahreszeitabhängig. Durch den erfindungsgemäßen Aufbau mit zwei separaten, über eine Dialyse-membran miteinander in Verbindung stehenden Kreisläufen wird es möglich, die optische Dichte im Reaktorkreislauf nahezu in Echtzeit an die einfallende Lichtmenge anzupassen und so für eine Reaktorproduktivität nahe am photosynthetischen Optimum zu sorgen.

Zweckmäßigerweise umfasst der Reaktorkreislauf einen Reaktor-Pufferbehälter. Ein solcher Pufferbehälter erhöht die Kapazität für Volumenverschiebungen und stattet so das System mit einer größeren Flexibilität und Anpassungsfähigkeit aus. Der Reaktor-Pufferbehälter kann gleichzeitig ein Entgasungs-Behälter sein. Ein separater Pufferbehälter ist jedoch ebenfalls möglich, Analog zum Reaktor-Pufferbehälter ist es vorteilhaft, wenn der Versorgungskreislauf ebenfalls einen Versorgungs-Pufferbehälter umfasst. Es werden dann größere Volumenverschiebungen zwischen dem Reaktorkreislauf und dem Versorgungskreislauf ermöglicht. Der Reaktor-Pufferbehälter kann eine Luft-Wasser-Gegenstromführung zur Entgasung aufweisen. Der die Dialysemembran beinhaltende Dialysator ist vorzugsweise ein Hohlfaserdialysator mit hoher spezifischer Membranfläche, der im Gegenstrombetrieb betrieben werden kann.

Der Versorgungskreislauf kann eine Oxidationseinheit umfassen. Die Hauptfunktion dieser Oxidationseinheit ist es, gelöste organische Endprodukte bzw. EOEP (Extrazelluläre Organische Endprodukte), die durch den Dialysator aus dem Photobioreaktor, also aus dem Reaktorkreislauf, ausgeschleust wurden, zu oxidieren. Dies ist wichtig, da diese organischen Endprodukte wachstumsinhibierend auf phototrophe Organismen wirken können. Die Oxidationseinheit bietet die Möglichkeit, EOEP im Dialysat zu oxidieren und aus diesem zu entfernen. Die daraus resultierende niedrige Konzentration von solchen organischen Endprodukten im Dialysat begünstigt dann die Abführung der EOEP aus dem Photobioreaktor. Geringe EOEP-Konzentrationen im Reaktormedium sind wiederum wichtig, um dem übermäßigen Wachstum von Pilzen und Bakterien vorzubeugen, Mittels der angesprochenen Oxidationseinheit und einer geeigneten Steuerung kann so der EOEP-Gehalt im Reaktorkreislauf gezielt eingestellt werden. Der Photobioreaktor ist also eine Quelle für inhibierende organische Substanz, abhängig von der Qualität des Speisewassers kann aber auch bereits darin Organik enthalten sein, die oxidiert werden muss, da es ansonsten zu inhibierenden Primär- und Sekundäreffekten kommen kann.

Um die Oxidation in der Oxidationseinheit zu ermöglichen, kann beispielsweise eine Flotation mit Ozon und Luft, eine Oxidation mit Wasserstoffperoxid und einem Katalysator oder eine biologische Oxidation mit Mikroben oder Enzymen durchgeführt werden. Eine Flotation bietet dabei den Vorteil, dass auch feinpartikuläre Substanz mit Teilchengrößen unter 20 µm ausgetragen werden und so einer Verblockung von Poren in der Dialyse-Membran entgegengewirkt werden kann. Um eine richtige Einstellung der Konzentration an Oxidantien zu erzielen, wird die Zufuhr der Oxidantien über eine Redoxpotential-Messung im Dialysat geregelt. Dies ist vorteilhaft, um den Durchbruch von Oxidantien in den Photobioreaktor zu vermeiden.

Eine bevorzugte Ausführungsform weist eine Hohlfasermembran als Dialysemembran auf. Um einen effektiven dialytischen Austausch zu ermöglichen, ist es vorteilhaft, wenn eine möglichst große Dialysefläche, also eine möglichst große Oberfläche der Dialysemembran, zur Verfügung steht. Weiterhin sind eine geringe Dicke der Dialysemembran sowie eine hohe Porosität von Vorteil.

Die Größe der Membran ist wesentlich von der spezifischen maximal erreichbaren Produktivität des Photobioreaktors und der Konzentration der limitierenden Komponente im Speisewasser abhängig. Die Bauweise entspricht der eines Hohlfaserdialysators. Für einen 1000l Photobioreaktor kann die Größe der Dialysemembran z.B. zwischen 10 m² und 60 m² liegen. Für eine effiziente Integration ist es vorteilhaft, wenn die Packungsdichte der Membran größer als 1000 m²/m³ ist. Um eine ausreichend hohe Stoffübertragungsleistung zu erzielen kann die Wandstärke der Membran zwischen 20-200µm liegen und/oder eine Porosität größer als 0,3 und/oder eine Tortuosität kleiner als 2,5 aufweisen. Wenn der Innendurchmesser der Fasern zwischen 100-400µm liegt kann eine effiziente Innenspülung durchgeführt werden. Abhängig von der Algenart und derer Exkretionsprodukten kann die Porengröße der Innenseite (lumenseitig) der Membran beispielsweise zwischen 8 und 200nm liegen. Bevorzugt wird eine Membran mit einer Porengröße zwischen 100nm und 200nm gewählt, um möglichst eine große Fraktion an extrazellulären organischen Endprodukten auszutragen und gleichzeitig Bakterien und größere Mikroorganismen vor den Eintritt in den Reaktorkreislauf zurück zu halten. Weiterhin kann die Stoffübertragungsleistung des Dialysators durch Veränderung der Strömungsgeschwindigkeit oder durch Einspeisung von Gasblasen im Faserlumen sowie im Mantelraum variiert werden.

Eine große Membran und somit ein effektiver Nährstoffaustausch zwischen dem Reaktorkreislauf und dem Versorgungskreislauf ermöglicht ebenfalls einen effektiven Wärmeaustausch zwischen den beiden Kreisläufen. Um ein Überhitzen des Reaktorkreislaufs bei starker Sonneneinstrahlung bzw. bei erhöhter Absorption zu vermeiden, kann in dem Versorgungskreislauf ein Wärmetauscher vorhanden sein. Wenn der Versorgungkreislauf einen Wärmetauscher umfasst, kann auf eine aufwendige Kühlung des Reaktorkreislaufes verzichtet werden. Üblicherweise werden solche Reaktorkreisläufe extern z. B. durch eine Besprenkelungseinheit gekühlt. Im Sommer und zur sonnigen Mittagszeit kann dem Photobioreaktor also dem Reaktorkreislauf, Wärme über den Dialysator abgeführt werden. In erster Linie wird dies durch die hohe Wärmekapazität des Dialysats erzielt. Optional kann dann der Wärmetauscher zugeschaltet werden, um Wärme über Kühlwasser abzuführen. Anstatt eines Wärmetauschers kann auch ein Kühlturm eingesetzt werden, um dem Dialysat direkt über Verdunstung Wärme zu entziehen. Im Winter oder bei Einsatz von thermophilen Organismen kann dem Reaktormedium über den Wärmetauscher Wärme zugeführt werden.

In dem Reaktorkreislauf kann eine Vorrichtung zum Einstellen des pH-Werts vorhanden sein. Dies kann durch Zufuhr von Kohlenstoffdioxid passieren. Es ist zwar möglich, einen Großteil des benötigten Kohlenstoffdioxids mittels hydrogencarbonathaltigem Speisewasser über die Dialyse zuzuführen, bei Schwankungen der Speisewasserqualität oder Schwankungen der solaren Einstrahlung muss jedoch das System in der Lage sein, schnell genug auf einen temporär erhöhten Kohlenstoffdioxidbedarf zu reagieren. Zu diesem Zweck kann reines Kohlenstoffdioxid oder Rauchgas zugeführt werden, beispielsweise auf der Saugseite der Reaktorpumpe.

Ähnlich wie bei klassischen Membranverfahren kommt es auch bei Dialysatoren nach längerer Betriebszeit zu Fouling. Um eine Reinigung des Dialysators vorzunehmen, kann der Reaktorkreislauf eine Spülpumpe umfassen. Mittels dieser Spülpumpe kann der Dialysator gespült und so gereinigt werden.

Die mechanische Spülung mittels der Spülpumpe kann zu einem unkontrollierten Transmembranfluss zwischen Dialysat und Reaktormedium führen. Dies kann zu erheblichen Füllstandschwankungen im Reaktorkreislauf und gegebenenfalls zu einer unkontrollierten Ernte, bildlich gesprochen also einem Überlaufen des Reaktorkreislaufs, führen. Abhängig von den Einstellungen der Außen- sowie der Innenspülung kann es sowohl zu einem Überlaufen als auch zu einem Leerlaufen des Reaktors kommen. Um dies zu vermeiden, kann der Reaktorkreislauf einen Füllstandsensor umfassen. Vorteilhafterweise erfasst der Füllstandsensor den Füllstand im Entgasungsbehälter. Basierend auf der Füllstandmessung kann die Leistung bzw. die Frequenz der Versorgungspumpe derart geregelt werden, dass der Sollfüllstand wieder erreicht wird. Der Füllstandssensor kann außer zum Vermeiden eines Überlaufens während der Spülung auch zur Bestimmung des Volumens im Reaktorkreislauf während der Regelung der optischen Dichte genutzt werden.

Am Reaktorkreislauf kann eine Entnahmevorrichtung vorhanden sein, die im Reaktorkreislauf enthaltenes Medium bei Überschreiten eines festgelegten Füllstand automatisch einer Auffangeinheit zuführt. Auf diese Art und Weise ist eine einfache Erntemöglichkeit vorhanden. Um einen Teil der Algensuspension zu entnehmen, muss lediglich das Volumen im Reaktorkreislauf erhöht werden. Andere Methoden zur Ernte, wie z. B. ein Ablassventil oder ähnliches sind natürlich ebenfalls denkbar.

Weiterhin kann im Reaktorkreislauf ein Sensor vorhanden sein, der die optische Dichte im Reaktor misst. Dieser Messwert kann für die Bestimmung des Sollwerts für das Volumen in dem Zuchtbehälter bzw. in dem Reaktorkreislauf verwendet werden. Beispielsweise können abhängig von der Jahreszeit für jede Tageszeit Sollwerte für die optische Dichte festgelegt und abgespeichert werden. Aus diesen lässt sich wiederum zusammen mit dem Messergebnis der aktuellen optischen Dichte ein Sollwert für das Volumen ermitteln. Über eine geeignete Steuerung der Reaktorpumpe und/oder der Versorgungspumpe kann ein entsprechender Nettofluss durch die Dialysemembran eingestellt werden, bis das gewünschte Volumen im Reaktorkreislauf erreicht ist. Ist der Sollwert des Volumens im Reaktorkreislauf erreicht, so werden die Leistungen bzw. Frequenzen der beiden Pumpen so aufeinander abgestimmt, dass die in dem Reaktorkreislauf und in dem Versorgungskreislauf vorhandenen Volumina konstant bleiben. Mit anderen Worten werden also auf Grundlage der aktuellen optischen Dichte, was ein Maß für die Algenkonzentration ist, und aus einem Kennwert für die aktuelle Sonneneinstrahlung ein Soll-Volumen für den Reaktorkreislauf bestimmt und eingestellt.

Der Versorgungskreislauf kann einen Vorfilter umfassen, der eine Porengröße von z. B. 20 µm aufweist. Größere organische oder anorganische Moleküle gelangen somit nicht bis zur Dialysemembran, wodurch eine zu starke Verschmutzung und in der Folge Zusetzung der Dialysemembran vermieden wird.

Im Folgenden wird anhand der Figuren ein Ausführungsbeispiel der Erfindung beschrieben. Es zeigen:
- Figur 1 -: eine Schemazeichnung einer erfindungsgemäßen Anlage;
- Figur 2 -: ein Diagramm, in dem jeweils ein typischer Verlauf der Biomassekonzentration sowie des Füllstands des Reaktorkreislaufs über einen Tag hinweg gezeigt ist;
- Figur 3 -: eine Darstellung der Abhängigkeit zwischen Produktivität und Biomassekonzentration bei zwei verschiedenen Lichtstärken;
- Figur 4 -: eine Ansicht von Versorgungs- und Reaktorkreislauf mit gesteuertem Flüssigkeitsaustausch;
- Figur 5 -: eine weitere Ansicht von Versorgungs- und Reaktorkreislauf mit gesteuertem Flüssigkeitsaustausch und
- Figur 6 -: eine Darstellung möglicher Schaltformen des erfindungsgemäßen Verfahrens.

Der Übersichtlichkeit halber sind nicht in allen Figuren alle Bauteile mit Bezugszeichen versehen. Gleiche Symbole bedeuten figurenübergreifend aber prinzipiell Bauteile gleicher Art, so dass ggf. die zu einer anderen Figur gehörenden Beschreibung eines Bauteils auf eine bestimmte Figur übertragen werden kann.

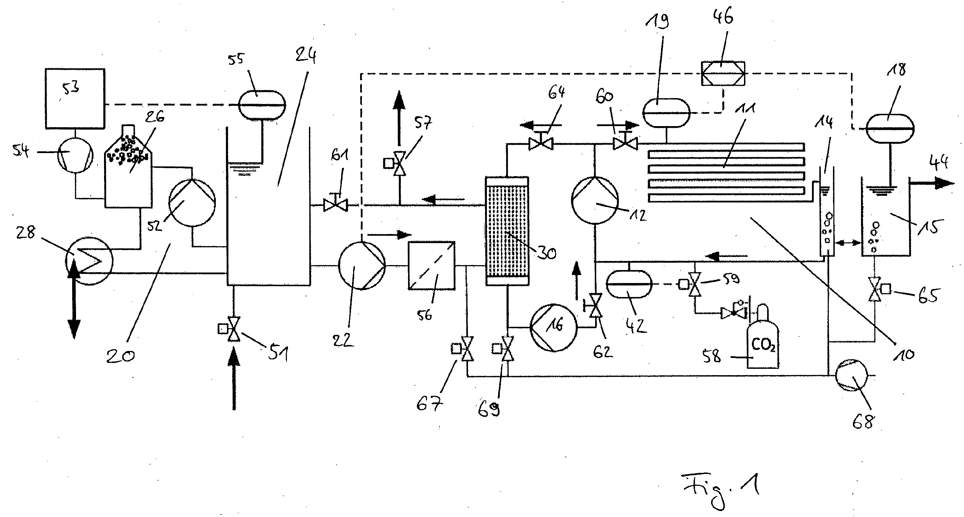

In Figur 1 ist ein Ausführungsbeispiel einer erfindungsgemäßen Anlage schematisch dargestellt. Der Reaktorkreislauf 10 ist im rechten Bereich der Figur und der Versorgungskreislauf 20 im linken Teil der Figur dargestellt. Pfeile geben Stoff- oder Wärmeströme an. Der Versorgungskreislauf 20 umfasst einen Versorgungs-Pufferbehälter 24, in den durch ein Ventil 51 Speisewasser geleitet werden kann. Das Speisewasser kann ein Prozesswasser, ein Abwasser oder ein Nährmedium sein. Unter einem Prozesswasser wird in diesem Zusammenhang beispielsweise Wasser verstanden, das bei der Produktion von Fischen oder anderen aquatischen Lebewesen in einer Aquakulturanlage anfällt. Die Versorgungspumpe 22 pumpt das Speisewasser aus dem Reaktor-Pufferbehälter 15 zur Dialysemembran 30. Der Pufferbehälter kann ein Volumen aufweisen, das zumindest so groß ist, wie das Volumen des Reaktorkerns. Vorzugsweise ist das Volumen des Pufferbehälters 15 zumindest um den Faktor 2 größer als das Volumen des Reaktorkerns 11.

Eine Aufbereitungspumpe 51 kann Speisewasser aus dem VersorgungsPufferbehälter 24 durch die Oxidationseinheit 26 und durch einen Wärmetauscher 28 pumpen. Im Anschluss fließt das Speisewasser wieder in den Versorgungs-Pufferbehälter 24 zurück. Im gezeigten Ausführungsbeispiel arbeitet die Oxidationseinheit 26 mit einem Ozongenerator 53 und einem Kompressor 54 zusammen. Der Ozongenerator 53 ist Redoxpotential-gesteuert, wobei das entsprechende Redoxpotential im Versorgungs-Pufferbehälter 24 von einem Sensor 55 gemessen wird. Der Kompressor 54 fördert das produzierte Ozon in die Oxidationseinheit 26, in der dann eine oxidative Flotation zum Austrag feiner Trübstoffe durchgeführt wird. Für die Oxidationseinheit 26 sind auch andere Funktionsweisen denkbar, so kann diese z. B. ein Oxidationsreaktor sein, ebenfalls kann der Ozongenerator 53 durch eine andere Quelle für Oxidantien wie z. B. Wasserstoffperoxid ersetzt werden. Eine biologische Oxidation mit Luftsauerstoff ist ebenfalls denkbar.

Der Wärmetauscher 28 kann für eine Temperierung des Speisewassers im Versorgungskreislauf und somit auch für eine Temperierung des Reaktorkreislaufs 10 genutzt werden. Das Ventil 51 dient zum Befüllen des Versorgungs-Pufferbehälters 24 wie auch zum Ablassen des Speisewassers. Auf diese Art und Weise ist es möglich, dass genutzte Speisewasser zyklisch auszutauschen. Bei sehr geringen Nährstoffkonzentrationen im Speisewasser ist ein kontinuierlicher Durchfluss durch den Versorgungskreislauf einstellbar. Dabei kann über das Ventil 51 Speisewasser zugeführt und über das Ventil 57 Dialysat abgeführt werden, wobei ein Nettodurchfluss an Speisewasser durch den Versorgungskreislauf kleiner ist als der Durchfluss an Dialysat durch den Mantelraum der Dialyse.

Im Betrieb wird Speisewasser in den Versorgungs-Pufferbehälter 24 eingeleitet. Das Speisewasser kann ein Prozess- oder ein Abwasser oder ein Nährmedium sein. Es kann anteilig sowohl partikuläre als auch gelöste Organik enthalten. Vom Versorgungs-Purferbehälter 24 fließt ein Teilstrom Richtung Dialysator 30 durch den Filter 56. Der Filter 56, der z. B. ein kontinuierlicher Trommelfilter oder ein Sandfilter sein kann, hat die Funktion, Partikel mit einem Durchmesser von z. B. mehr als 20 µm aus dem System zu entfernen. Selbstverständlich können auch andere Porengrößen gewählt werden. So sind beispielsweise Porengrößen von maximal 5 µm, maximal 10 µm oder maximal 40 µm denkbar. Es kann so einer Verblockung im Mantelraum des dichtgepackten Hohlfaser-Dialysators 30 entgegengewirkt und eine optimale Strömungsverteilung erhalten werden. Die aus dem Dialysator 30 ausfließende Dialysat-Lösung kann entweder über das Ventil 57 aus dem System entfernt werden oder in den Versorgungs-Pufferbehälter bzw. Verteilertank 24 zurückgeleitet werden. Um eine hohe Abreicherung an anorganischen Nährstoffen der Dialysat-Lösung zu erreichen, ist eine "Fed-Batch-Betriebsweise" für den Dialysator-Kreislauf vorteilhaft. Dabei wird ein zyklischer Wasseraustausch bei Erreichen eines definierten Minimalwerts der limitierten Nährstoffkomponente durchgeführt. Dazu wird das Dialysat im ersten Schritt bis zu einem Minimalstand im Puffertank 24 abgeführt und erst dann durch die Zufuhr von neuem Speisewasser substituiert.

Ein zweiter Teilstrom wird vom Versorgungs-Pufferbehälter 24 in die Oxidationsstufe 26 geleitet. In der Oxidationseinheit 26 werden gelöste organische Endprodukte, die durch den Dialysator aus dem Reaktorkreislauf 10 ausgeschleust wurden, oxidiert. Dies ist wichtig, da diese organischen Endprodukte auch wachstumsinhibierend auf phototrophe Organismen wirken können, Ein typisches Endprodukt von photoautotrophen Mikroorganismen sind EOEP, Ein weiteres inhibierendes Endprodukt von Mikroalgen und Cyanobakterien sind Polysaccharide Dementsprechend ist die Oxidationsstufe 26 besonders wichtig, da sie EOEP im Dialysat oxidiert und die Abführung von EOEP aus dem Reaktorkreislauf 10 durch niedrige Konzentrationen im Dialysat begünstigt wird. Geringe EOEP-Konzentrationen im Reaktormedium sind allerdings wichtig, um dem übermäßigen Wachstum von Pilzen und Bakterien vorzubeugen. Der Sensor 55 misst das Redoxpotential im Versorgungs-Pufferbehälter 24. Auf Basis dieser Messungen werden dann der Ozongenerator 53 und der Kompressor 54 gesteuert, um eine Flotation sowie Oxidation in der Oxidationseinheit 26 durchzuführen.

Im Reaktorkreislauf 10 wird ausgehend von der zentralen Reaktorpumpe 12 der Hauptstrom an Reaktormedium durch den photosynthetisch aktiven Reaktorkern 11 geleitet. Im Entgasungsbehälter 14, wird nach einem Gegenstromverfahren Sauerstoff durch Begasung mit Luft ausgetragen. Ein Pufferbehälter 15 ermöglicht es, das Volumen und somit die optische Dichte im Reaktorkreislauf 10 einzustellen. Die optische Dichte wird dabei von einem Sensor 19 gemessen. Weiterhin wird der pH-Wert über die Zufuhr von Kohlenstoffdioxid geregelt. Dabei wird von einem Sensor 42 der pH-Wert im Reaktorkreislauf 10 gemessen und auf Basis dieser Messdaten kontrolliert über ein Ventil 59 Kohlenstoffdioxid, das beispielsweise in einer Gasflasche 58 bereitstehen kann, zugeführt. Die Zugabe des Kohlenstoffdioxids erfolgt dabei auf der Saugseite der Reaktorpumpe 12. Statt Kohlendioxid kann z. B. auch Rauchgas zugeführt werden.

Ein zweiter Teilstrom wird mittels der Reaktorpumpe 12 durch das Ventil 64 in der Figur nach links in Richtung des Dialysators 30 geleitet. Die Reaktorpumpe 12 wirkt also gleichzeitig als Zirkulationspumpe für den Photobioreaktor 11 und als Zulaufpumpe für das Faserlumen des Dialysators 30. Das Verhältnis der beiden Teilströme in den Reaktorbehälter 11 und durch den Dialysator 30 kann z. B, durch die Ventile 64 und 60 eingestellt werden. Für weitere Einstellungsmöglichkeiten der Druckverhältnisse am Dialysator 30 stehen zusätzlich die Ventile 61 und 62 zur Verfügung. Im Dialysator 30 können Nährstoffe aus dem Versorgungskreislauf 20 in den Reaktorkreislauf 10 übertreten, wobei unerwünschte Bestandteile des Versorgungsmediums durch die Poren der Dialysemembran 30 zurückgehalten werden. Die Dialysemembran 30 wirkt somit als Hygienefilter. Kleine Teilchen im Reaktorkreislauf 11, wie z. B. Stoffwechselendprodukte und unerwünschte Sekundärmetabolite können durch die Dialysemembran 30 in den Versorgungskreislauf 10 übertreten und von dort aus entsorgt werden. Die Algen werden dabei von der Dialysemembran 30 zurückgehalten, da sie im Regelfall eine Teilchengröße aufweisen, die größer als die Poren der Dialysemembran ist. Nach dem Verlassen des Dialysators 30 fließt der angesprochene Teilstrom rückwärts durch die Spülpumpe 16 und durch ein weiteres Ventil 62 zur Reaktorpumpe 12 zurück.

Nach längerer Betriebszeit kann es im Dialysator 30 zu Fouling kommen. Um diesem effektiv vorzubeugen, ist in der dargestellten Anlage 2 ein automatisiertes Spülverfahren vorgesehen. Dabei wird eine zyklische Außenspülung im Mantelraum des Dialysators und eine zyklische Innenspülung im Faserlumen des Dialysators durchgeführt. Bei beiden Spültypen handelt es sich um eine Zwei-Phasen-Spülung aus Luft und Wasser. Für die Außenspülung mittels des Dialysatkreislaufs bzw. des Versorgungskreislaufes 20 wird in diesem die Strömungsgeschwindigkeit mittels der Versorgungspumpe 22 erhöht und gleichzeitig mittels eines Kompressors 68 über das Magnetventil 67 mit Luft begast. Dies führt zu einem Austrag von äußerem Biofilm und Organik. Für die Innenspülung auf der Reaktorseite wird zyklisch die Strömung im Dialysat 30 umgekehrt indem die Reaktorpumpe 12 und die Spülpumpe 16 abwechselnd betrieben werden. Zusätzlich wird das Faserlumen mit Luft begast. Dies geschieht wiederum mittels des Kompressors 68 in Verbindung mit dem Magnetventil 69. Die Innenspülung führt vor allem dazu, dass Poren in der Membran 30 gespült werden und einer Kuchenbildung von Algen im Einlaufbereich des Dialysators entgegengewirkt wird. Es werden primär die Fasern freigehalten, so dass eine gleichmäßige Strömung über die Dialysemembran erhalten bleibt. Sekundär werden auch die Poren in der Membran gespült. Durch die regelmäßige mechanische Spülung des Dialysators wird es möglich, lange Betriebszyklen zu erreichen.

In Figur 1 wird deutlich, dass Entgasungsbehälter 14 und Pufferbehälter 15 im Reaktorkreislauf voneinander entkoppelt sind. Dies ist vorteilhaft, da dann der Entgasungsbehälter 14 deutlich kleiner als der Pufferbehälter 15 dimensioniert werden kann. Bevorzugt beträgt das Volumen des Entgasungsbehälters 14 abhängig von der Reaktorgeometrie etwa 5-20% des photosynthetisch aktiven Reaktorvolumens des Reaktorkerns 11. Der Pufferbehälter 15 weist vorteilhafterweise eine Größe von mindestens 200% dieses Volumens auf. Es kann dann eine optimale Echtzeit-Regelung der optischen Dichte realisiert werden. Eine Kombination von Puffer- und Entgasungsbehälter als ein Bauteil ist ebenfalls denkbar, das Aufteilen in zwei separate Behälter bietet jedoch einige Vorteile. So ist mit getrennten Behältern eine hocheffiziente Entgasung, für die ein relativ kleiner Behälter benötigt wird, gleichzeitig mit einer ausreichenden Pufferfunktion realisierbar, die einen möglichst großen Behälter benötigt. Weiterhin wird durch ein Verkleinern des Entgasungsbehälters 14 der kontinuierlich durchströmter Dunkelraum verkleinert, was die photosynthetische Effizienz des Gesamtverfahrens gegenüber eines kombinierten Entgasungs- und Pufferbehälters deutlich erhöht.

Um eine Sedimentation im strömungsberuhigten Pufferbehälter 15 des Reaktokreislaufs zu vermeiden, besteht die Möglichkeit über die Ansteuerung von Ventil 65 zyklisch Luft einzugasen. Jegliche Luft die in den Reaktorkreislauf 10 eingespeist wird, muss über einen Luftfilter sterilisiert werden. Luftfilter wurden aus Gründen der Übersichtlichkeit nicht eingezeichnet.

Bei der mechanischen Spülung kann es zu einem unkontrollierten Transmembranfluss zwischen Dialysat und Reaktormedium kommen. Dies kann zu Füllstandschwankungen im Entgasungsbehälter 14 und im schlimmsten Fall zu einer unkontrollierten Ernte führen. Um dies zu vermeiden, wird der Füllstand im Entgasungsbehälter 14 mittels des Sensor 18 erfasst. Basierend auf der Füllstandmessung im Entgasungsbehälter 14 wird die Frequenz der Versorgungspumpe 22 so lange verändert, bis der Sollwert für den Füllstand wieder erreicht wird, Durch eine schrittweise Änderung der Pumpenfrequenz werden die Druckverhältnisse im Dialysator 30 wieder so eingestellt, dass der Netto-Transmembranfluss gleich Null ist und der Soll-Füllstand konstant bleibt.

Die Füllstandregelung ist mit einer Regelung der optischen Dichte verknüpft. Die optische Dichte ist wiederum eine Funktion der Biomassekonzentration und wird im Reaktorkern 11 gemessen. Abhängig von dem Sonnenstand und der Jahreszeit gibt es einen Sollwert für die optische Dichte bzw. Biomassekonzentration. Das bedeutet, dass morgens und abends entsprechend dem Sonnenstand niedrige und mittags hohe Biomassekonzentrationen im Reaktor eingestellt werden, um das photosynthetische Optimum zu erreichen. Die Biomassekonzentration ist eine Funktion des Füllstands. Sie lässt sich also durch Volumenzufuhr von Flüssigkeit einstellen. Durch Änderung der Frequenz der Versorgungspumpe 22 lassen sich die Druckverhältnisse im Dialysator 30 und somit der Füllstand im Reaktor und damit im Entgasungsbehälter 14 einstellen. Ausgehend von einem normalen Füllstand im Entgasungsbehälter 14 führt eine Absenkung des Soll-Füllstands zu einer Aufkonzentrierung der Biomasse. Eine Erhöhung des Soll-Füllstands führt zu einer Verdünnung der Biomasse. Solange der Füllstand die Höhe eines Ernteablaufs, über den automatisch Algensuspension geerntet werden kann, nicht erreicht wird und Suspension ausläuft, ist diese Verdünnung reversibel.

Die Steuerung der optischen Dichte wird also über die Veränderung des Sollfüllstands im Entgasungsbehälter 14 realisiert. Die optische Dichte wird direkt von dem Sensor 19 im Reaktorkern 11 erfasst und dann von einer Recheneinheit in ein Soll-Follstandsignal umgerechnet, mit welchem dann die Versorgungspumpe 22 geregelt wird. Dazu wird die aktuell gemessene optische Dichte (OD) mit dem Sollwert der optischen Dichte, welcher eine mathematische Funktion der aktuellen Lichtstärke ist, verglichen. Weicht der aktuelle OD-Wert vom OD-Sollwert abzgl. einer Toleranz ab, so wird berechnet, wie viel Volumen zu- oder abgeführt werden soll, um den OD-Sollwert zu erzielen. Aus dem entsprechenden Volumenwert wird über eine geometrische Umrechnung ein Füllstandsintervall berechnet. Eine Volumenzunahme bzw. Verdünnung entspricht einem positiven Füllstandsintervall, eine Volumenabnahme bzw, Aufkonzentrierung entspricht einem negativen Füllstandsintervall. Der neue Sollfüllstand ergibt sich aus der Addition des berechneten Füllstandsintervalls und dem aktuell gemessenen Füllstand im Pufferbehälter 15, gemessen durch den Füllstandssensor 19. Das Soll-Follstandssignal dient zur Regelung der Versorgungspumpe 22.

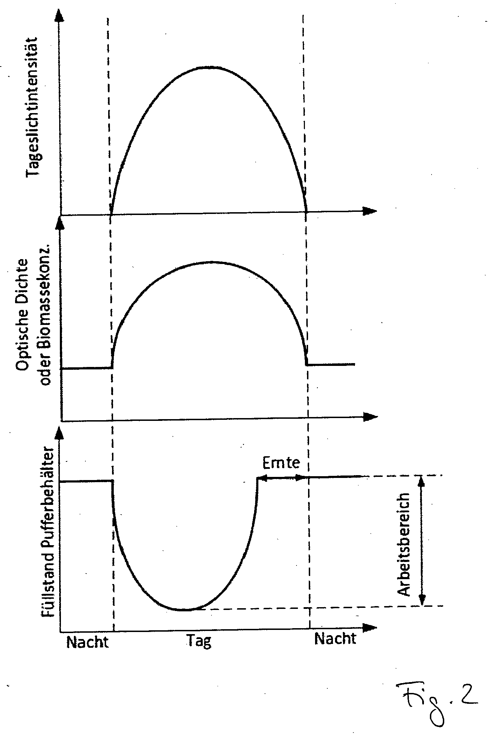

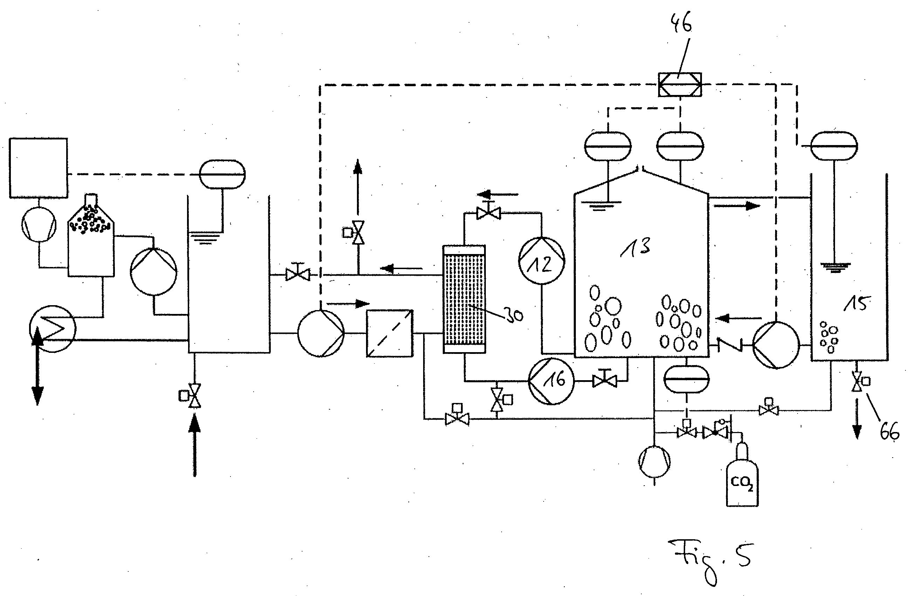

In Figur 2 ist der Zusammenhang zwischen Biomassekonzentration, optischer Dichte und Füllstand sowie der Intensität der Sonneneinstrahlung visualisiert. Auf der x-Achse ist dabei die Zeit aufgetragen. Auf der y-Achse ist im unteren Bereich der Figur der Füllstand, im mittleren Bereich der Figur die optische Dichte der Biomassekonzentration und im oberen Bereich der Figur die Tageslichtintensität als Maß für die Biomassekonzentration im Verlauf eines Tages dargestellt. Es sind dabei Sollkurven in Abhängigkeit vom Tageszyklus dargestellt. Die Sollkurven variieren dabei von Tag zu Tag, da sie von der Tageslänge und der Lichtstärke abhängig sind. Die Sollkurve für die optische Dichte definiert dabei die Sollkurve für den Füllstand im Entgasungsbehälter 14 des Reaktorkreislaufs 10. Im dargestellten Beispiel ist die Sollkurve für den Füllstand zeitlich etwas versetzt zu der Sollkurve für die optische Dichte, da es im Tagesverlauf zusätzlich zu Wachstum von Biomasse kommt. Dies führt weiterhin dazu, dass nachmittags Lösung aus dem Reaktor durch gezieltes Überschreiten des Maximalfüllstands geerntet wird. Das entnommene Volumen wird durch frisches Speisewasser ersetzt. Weiterhin wird durch diese Maßnahmen sichergestellt, dass am nächsten Tag im vorgegebenen Arbeitsbereich wieder mit einer niedrigen optischen Dichte gestartet werden kann. Der eingezeichnete Arbeitsbereich ist als das Füllstandintervall zwischen dem Überlauf und dem für die Pumpe minimal zulässigen Füllstand definiert. Der Arbeitsbereich ist somit von Größe und Geometrie des Entgasungsbehälters abhängig. Weiterhin lässt sich die Füllstandregelung auch auf Reaktortypen anwenden, bei denen Entgasungsbehälter und photosynthetisch aktiver Reaktorkern als eine Einheit realisiert sind, wie z.B. bei einem Flachplatten-Reaktor. (s. hierzu auch Fig. 5).

Das entwickelte Kreislaufverfahren ist hervorragend geeignet für die Integration in eine kreislaufgeführte Aquakultur-Anlage. Der Versorgungs-Pufferbehäfiter 24 kann dabei durch ein Fischbecken ersetzt werden und mit weiteren Aufbereitungsvorrichtungen, wie z. B. einem Biofilter, ergänzt werden. Dabei würde der Reaktorkreislauf 10 die Funktion einer Denitrifikationsanlage substituieren bzw. ergänzen.

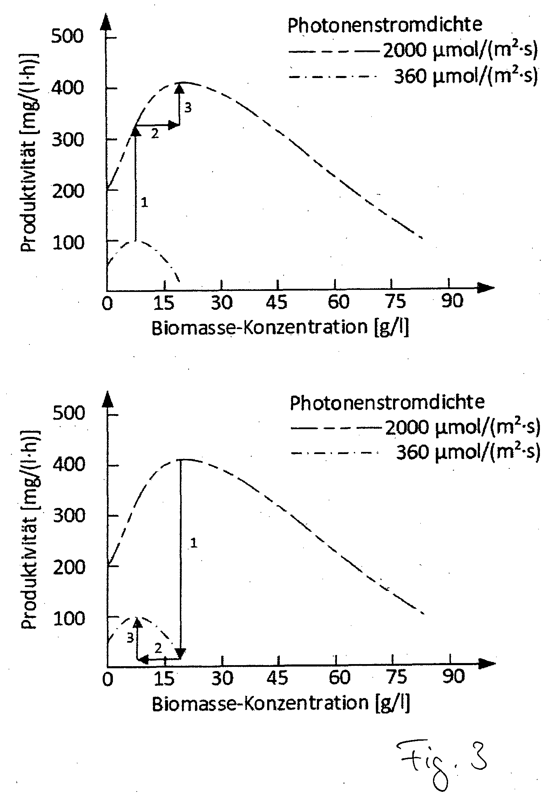

In Figur 3 ist die Abhängigkeit zwischen Produktivität und Biomassekonzentration bei zwei verschiedenen Lichtstärken in Anlehnung an Daten zu Chlorococcum littorale von Hu et al., "Ultrahigh-cell-density culture of a marine green alga Chlorococcum littorale in a flat-plate photobioreactor", Appl Microbiol Biotechnol 49, 655-662, dargestellt. Dabei ist im oberen Bereich der Figur eine schematische Regel-Strecke für das Zeitintervall zwischen einem Zeitpunkt kurz nach Sonnenaufgang und der Mittagszeit an einem wolkenfreien Sommertag gezeigt, und im unteren Bereich der Figur eine schematische Regel-Strecke für das Zeitintervall zwischen der Mittagszeit und einem Zeitpunkt kurz vor Sonnenuntergang an einem wolkenfreien Sommertag. In Figur 3 wird deutlich, dass die optimale Produktivität bei ausreichender Nährstoffversorgung und gegebener Reaktorgeometrie sowie gleichbleibender Temperatur im Wesentlichen von der Biomassekonzentration und der Lichtstärke abhängt. So wird bei einer Photonenstromdichte von 360 µmol/(m²·s) im Fall von Chlorococcum litorale in einem Flachplattenreaktor (1cm Dicke) bei 25°C eine optimale Produktivität bei etwa 8 g/l erreicht, während bei einer Photonenstromdichte von 2000 µmol/(m²·s) die optimale Produktivität erst bei etwa 17 g/l Biomassekonzentration erreicht wird. Das erfindungsgemäße Verfahren macht sich genau diesen biophysikalischen Zusammenhang zum Nutzen. Dies lässt sich an zwei Fallbeispielen erläutern:
1. Intervall "Vormittags": Man betrachte das obere Diagramm in Fig.3 und nehme an, dass die Photonenstromdichte von 360 µmol/(m²·s) einer Zeit kurz nach Sonnenaufgang an einem sonnigen Sommertag entspricht. Ausgehend von der Programmierung des Rechners (in den Figuren mit dem Bezugszeichen 46 gekennzeichnet) ist die Biomassekonzentration bereits auf 8 g/l eingestellt, sodass eine optimale Produktivität erzielt wird. Nun steigt der Sonnenstand, sodass zur Mittagszeit eine maximale Photonenstromdichte von 2000 µmol/(m²·s) erreicht wird (Linie 1). Da die Änderung der Biomassekonzentration durch Wachstum in so einem Zeitintervall vernachlässigbar klein ist, würde ohne aktive Änderung die Produktivität zur Mittagszeit bei etwa 320 mg/(l·h) liegen. Dies liegt unterhalb des Optimums. Durch die Regelung wird jedoch im Verlauf der Vormittagszeit kontinuierlich eine Konzentration von 17 g/l eingestellt (Linie 2), sodass eine optimale Produktivität von 410 mg/(l·h) erzielt wird (Linie 3). Ausgehend von einem 1m³ Photobioreaktor müssen dazu über die Dialyse 0.53m³ algenfreie Lösung abgesaugt werden. Die fehlende Lösung fließt in Form von Algensuspension aus dem Pufferbehälter nach. In diesem Beispiel ergibt sich durch die Regelung folgende Effizienzsteigerung: (1-410/320)*100%= 28% Die Produktivität zur Mittagszeit wird also um 28 % durch die Echtzeit-Regelung gesteigert.
2. Intervall "Nachmittags": Man betrachte das obere Diagramm in Fig.3 und nehme an, dass die Photonenstromdichte von 2000 µmol/(m²·s) der Mittagszeit an einem sonnigen Sommertag entspricht. Ausgehend von der Programmierung des Rechners (SPS) ist die Biomassekonzentration bereits auf 17 g/l eingestellt, sodass eine optimale Produktivität erzielt wird. Nun sinkt der Sonnenstand, sodass kurz vor Sonnenuntergang eine Photonenstromdichte von 360 µmol/(m²·s) erreicht wird (Linie 1). Da die Änderung der Biomassekonzentration durch Wachstum in so einem Zeitintervall vernachlässigbar klein ist, würde ohne aktive Änderung die Produktivität kurz vor Sonnenuntergang bei etwa 10 mg/(l·h) liegen. Dies liegt unterhalb des Optimums. Durch die Regelung wird jedoch im Verlauf der Nachmittagszeit kontinuierlich eine Konzentration von 8 g/l eingestellt (Linie 2), sodass eine optimale Produktivität von 100 mg/(l·h) erzielt wird (Linie 3). Ausgehend von einem 1m³ Photobioreaktor müssen dazu über die Dialyse 1,13 m³ algenfreie Lösung zugeführt werden. Die überschüssige Lösung fließt in Form von Algensuspension in den Pufferbehälter des Reaktorkreislaufs. In diesem Beispiel ergibt sich durch die Regelung folgende Effizienzsteigerung: (1-100/10)*100%= 900%. Die Produktivität zur Abendzeit wird also um 900% durch die Echtzeit-Regelung gesteigert. Weiterhin wird an diesem Beispiel deutlich, dass das Volumen des Pufferbehälters im Versorgungskreislauf genauso wie im Reaktorkreislauf größer sein muss, als das Volumen des photosynthetisch aktiven Photobioreaktors. Um einen ausreichenden Arbeitsbereich in der Regelung zu haben, sollte das Puffervolumen in beiden Kreisläufen zumindest zweimal so groß wie das Volumen des Photobioreaktors sein.

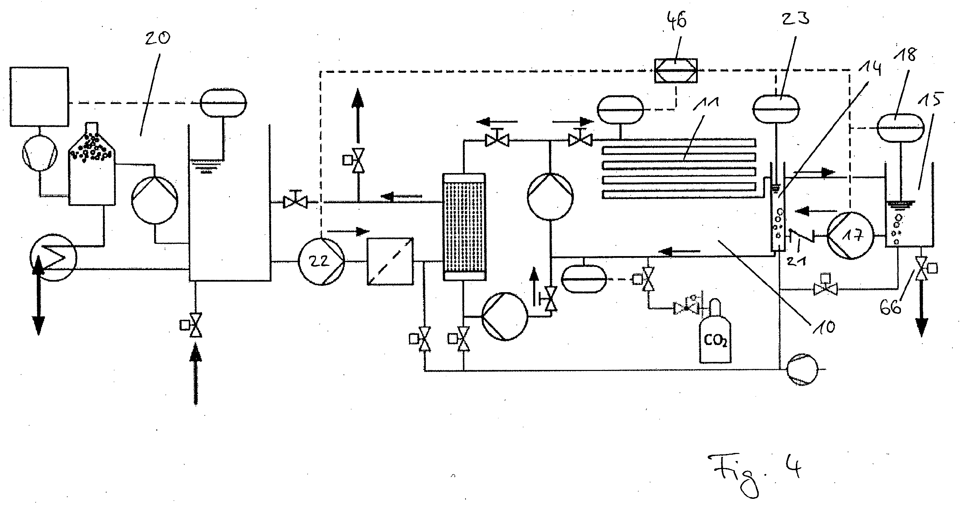

Figur 4 zeigt eine Ansicht von Versorgungskreislauf 20 und Reaktorkreislauf 10 mit gesteuertem Flüssigkeitsaustausch zwischen Entgasungsbehälter 14 und Pufferbehälter 15. Gezeigt ist eine weitere Variante der Verschaltung zwischen einem tubulären Photobioreaktor 11 und dem Pufferbehälter 15 dargestellt. Das Soll-Niveau im Entgasungsbehälter 14 bleibt hier immer nahezu gleich. Es wird so eingestellt, dass es kurz vorm Überlaufen in den Behälter 15 ist. Wie bereits beschrieben sorgt eine Füllstandregelung dafür, dass dieses Niveau durch Spülvorgänge unbeeinflusst bleibt. Soll nun die optische Dichte lichtabhängig erhöht werden, wird die Frequenz der Versorgungspumpe 22 reduziert, so dass algenfreie Lösung aus dem Reaktorkreislauf 10 in den Versorgungskreislauf 20 fließt. Gleichzeitig wird die Pumpe 17 angesteuert, sodass aktiv Algensuspension vom Pufferbehälter 15 in den Reaktorkreislauf 10 nachgepumpt wird. Hierbei bleibt das Niveau im Entgasungsbehälter 14 nahezu konstant, Der Füllstand im Entgasungsbehälter 14 wird durch einen Füllstandssensor 23 überwacht. Bei Erreichen des Sollwerts der optischen Dichte wird die Pumpe 22 wieder in den Ursprungszustand geregelt und die Pumpe 17 ausgeschaltet. Da hinter der Pumpe 17 das Rückschlagventil 21 ist, kann über diese Leitung keine Algensuspension vom Reaktorkreislauf 10 in den Pufferbehälter 15 zurückfließen. Soll nun die optische Dichte im Reaktorkreislauf 10 lichtabhängig verringert werden, wird die Frequenz der Pumpe 22 erhöht, sodass Lösung durch die Dialyse 30 in den Reaktor 11 fließt. Die Pumpe 17 bleibt ausgeschaltet. Dies führt nun dazu, dass der Entgasungsbehälter 14 aktiv überläuft bzw. Lösung aus dem Entgasungsbehälter 14 in den Pufferbehälter 15 fließt. Analog zur Verschaltung in Fig.1 und dem Diagramm in Fig. 2 wird auch hier der lichtabhängige Sollwert für die optische Dichte in einen Sollwert für den Füllstand im Pufferbehälter 15 umgerechnet. Weiterhin ist einerseits wie in Fig.1 die Ernte über einen freien Überlauf 44 (in Fig. 1 nur angedeutet) oder über einen gesteuerten Abzug mit dem Ventil 66 realisierbar.

In Figur 5 ist eine weitere Ansicht von Versorgungs- und Reaktorkreislauf mit gesteuertem Flüssigkeitsaustausch zwischen Flachplattenreaktor 11 und Pufferbehälter 15 gezeigt. Analog zur Verschaltung in Figur 4 ist das Verfahren in Kombination mit einem Flachplatten-Photobioreaktor 13 dargestellt. Das Besondere am Flachplatten-Photobioreaktor 13 ist, dass Entgasungsraum und photosynthetisch aktives Reaktorvolumen in einem Modul realisiert sind. Dies hat zur Folge, dass das Niveau im Reaktor 13 konstant bleiben muss bzw. nur in einer sehr kleinen Amplitude schwanken darf. Ein übermäßiges Absenken des Niveaus im Flachplattenreaktor 13 führt zwangsläufig zu einer Abnahme des nutzbaren Reaktorvolumens und somit zum Verlust von Effizienz und absoluter Produktionsleistung. Weiterhin fällt die Zirkulationspumpe 12 im Vergleich zum tubulären Reaktor deutlich kleiner aus, da sie nur den verhältnismäßig kleinen Kreislaufstrom durch das Faserlumen der Dialyse 30 fördern muss. Bei der mechanischen Rückspülung des Faserlumens wird Pumpe 12 aus- und Pumpe 16 eingeschaltet, Die interne Zirkulation des Flachplattenreaktors 11 wird durch die Begasung übernommen.

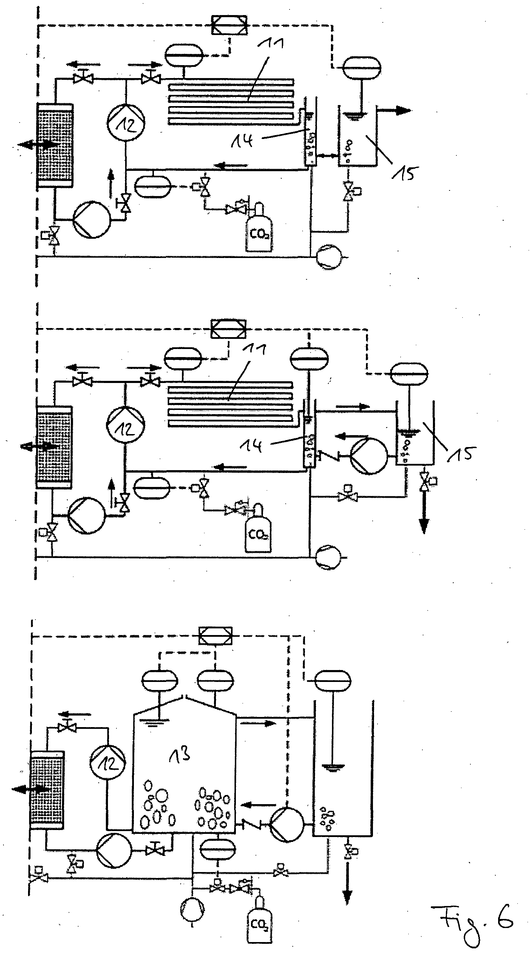

Figur 6 zeigt eine Darstellung möglicher Schaltformen des erfindungsgemäßen Verfahrens. Ausgehend von Fig. 6 stellt sich heraus, dass es zwei grundsätzliche Schaltformen gibt, den freien sowie den gesteuerten Flüssigkeitsaustausch zwischen Photobioreaktor 11 und Pufferbehälter 15. Der gesteuerte Flüssigkeitsaustausch ist Voraussetzung, um überhaupt einen Flachplatten-Photobioreaktor 13 zu integrieren. Der weitere große Vorteil des gesteuerten Austauschs in Kombination mit dem tubulären Photobioreaktor 11 ist die optimale Nutzung des Entgasungsbehälters 14 sowie ein minimaler Energieaufwand, da die Zirkulationspumpe 12 einen minimalen Höhenunterschied überwinden muss. Der Vorteil des freien Flüssigkeitsaustauschs ist der geringere Automatisierungsaufwand und der damit reduzierte Bedarf an Mess- und Regeltechnik sowie Apparaten.

### Bezugszeichenliste

- 2: Anlage
- 10: Reaktorkreislauf
- 11: Zuchtbehälter/Reaktorkern
- 12: Reaktorpumpe
- 13: Flachplatten-Photobioreaktor
- 14: Entgasungsbehälter
- 15: Reaktor-Pufferbehälter
- 16: Spülpumpe
- 17: Pumpe
- 18: Füllstandssensor
- 19: Sensor
- 20: Versorgungskreislauf
- 21: Rückschlagventil
- 22: Versorgungspumpe
- 23: Füllstandssensor
- 24: Versorgungs-Pufferbehälter
- 26: Oxidationseinheit
- 28: Wärmetauscher
- 30: Dialysemembran
- 42: Sensor
- 44: Auffangeinheit
- 46: Rechner
- 51: Ventil
- 52: Aufbereitungspumpe
- 53: Ozongenerator
- 54: Kompressor
- 55: Sensor
- 56: Filter
- 57: Ventil
- 58: Gasflasche
- 59: Ventil
- 60: Ventil
- 61: Ventil
- 62: Ventil
- 64: Ventil
- 65: Ventil
- 66: Ventil
- 67: Ventil
- 68: Kompressor
- 69: Ventil

## Patentansprüche

1. Anlage (2) zur Zucht von phototrophen Organismen, umfassend
a. einen Reaktorkreislauf (10) mit einer Reaktorpumpe (12),
b. einen Versorgungskreislauf (20) mit einer Versorgungspumpe (22) zum Aufbereiten einer Versorgungsflüssigkeit (24) und zum Versorgen des Reaktorkreislaufs (20) mit der Versorgungsflüssigkeit (24), und
c. eine Dialysemembran (30), die den Versorgungskreislauf (20) von dem Reaktorkreislauf (10) trennt, aber einen diffusiven, durch eine Porengröße der Dialysemembran (30) begrenzten Stoffaustausch erlaubt.

2. Anlage nach Anspruch 1, **dadurch gekennzeichnet, dass** der Reaktorkreislauf (10) einen Reaktor-Pufferbehälter (14) und der Versorgungskreislauf (20) einen Versorgungs-Pufferbehälter (24) umfasst, um Volumenverschiebungen zwischen dem Reaktorkreislauf (10) und dem Versorgungskreislauf (20) zu ermöglichen.

3. Anlage nach Anspruch 1 oder 2, **dadurch gekennzeichnet, dass** der Versorgungskreislauf (20) eine Oxidationseinheit (26) umfasst.

4. Anlage nach einem der vorstehenden Ansprüche, **dadurch gekennzeichnet, dass** die Dialysemembran (30) eine Hohlfasermembran ist.

5. Anlage nach einem der vorstehenden Ansprüche, **dadurch gekennzeichnet, dass** der Versorgungskreislauf (10) einen Wärmetauscher (28) umfasst.

6. Anlage (2) nach einem der vorstehenden Ansprüche, **dadurch gekennzeichnet, dass** der Reaktorkreislauf (10) eine Spülpumpe (16) umfasst.

7. Anlage (2) nach einem der vorstehenden Ansprüche, **dadurch gekennzeichnet, dass** der Reaktorkreislauf (10) einen Füllstandssensor (18) umfasst

8. Anlage (2) nach einem der vorstehenden Ansprüche, **dadurch gekennzeichnet, dass** am Reaktorkreislauf eine Entnahmevorrichtung vorhanden ist, die enthaltenes Medium bei Überschreiten eines festgelegten Füllstands automatisch einer Auffangeinheit (44) zuführt.

9. Verfahren zum Steuern einer Anlage zur Produktion von phototrophen Organismen mit den Schritten:
a. Bestimmen eines Kennwerts für eine auf einen Reaktor (10) zur Zucht von phototrophen Organismen einfallende Lichtmenge
b. Bestimmen eines Sollwerts für ein Volumen in einem Zuchtbehälter (11) unter Einbeziehung des Kennwerts für die einfallende Lichtmenge und/oder der Uhrzeit
c. Anpassen einer Pumpleistung einer Reaktorpumpe (12) und/oder einer Versorgungspumpe (22) derart, dass der Sollwert des Volumens im Reaktor eingestellt wird.

10. Verfahren nach Anspruch 9, **dadurch gekennzeichnet, dass** die optische Dichte im Reaktor gemessen und für die Bestimmung des Sollwerts für das Volumen verwendet wird.
